# EUROPÄISCHE PATENTSCHRIFT

(11) **EP 1 546 091 B1**
(45) Veröffentlichungstag und Bekanntmachung des Hinweises auf die Patenterteilung: **01.12.2010**
(21) Anmeldenummer: 03769284.5
(22) Anmeldetag: 18.09.2003
(51) Int. Cl.: C07C 263/20, C07C 265/14, B01D 3/00

(54) **AUFARBEITUNG VON REAKTIONSAUSTRÄGEN AUS PHOSGENIERREAKTOREN**
REPROCESSING OF REACTION MATERIAL DISCHARGED FROM PHOSGENATION REACTORS
TRAITEMENT DE PRODUITS REACTIONNELS ISSUS DE REACTEURS DE PHOSGENATION

(30) Priorität: 27.09.2002 DE 10245584
(43) Veröffentlichungstag der Anmeldung: 29.06.2005
(73) Patentinhaber: BASF SE, 67056 Ludwigshafen (DE)
(72) Erfinder: STRÖFER, Eckhard, 68163 Mannheim (DE); SCHOLL, Stephan, 38116 Braunschweig (DE); SOHN, Martin, 68229 Mannheim (DE); WÖLFERT, Andreas, 74906 Bad Rappenau (DE); PALLASCH, Häns-Jürgen, 67169 Kallstadt (DE); BREDEHÖFT, Jan, Peter, 67459 Böhl-Iggelheim (DE)
(86) Internationale Anmeldenummer: PCT/EP2003/010381
(87) Internationale Veröffentlichungsnummer: WO 2004/031132

(56) Entgegenhaltungen:
- FR-A- 1 487 546
- GB-A- 1 083 910
- US-A- 3 471 543
- US-A- 3 892 634
- US-A- 4 118 286
- US-A- 5 962 728
- DATABASE WPI Section Ch, Week 197708 Derwent Publications Ltd., London, GB; Class E14, AN 1977-001516 XP002269597 & RO 61 695 A (PETRU PONI INST CHI), 15. September 1976 (1976-09-15)

## Beschreibung

Die Erfindung betrifft ein Verfahren zur Herstellung von Isocyanaten durch Umsetzung von primären Aminen mit Phosgen in einem Reaktor, wobei der Reaktionsaustrag in Form einer Suspension, die das herzustellende Isocyanat als Flüssigkeit und Carbamylchloride als Feststoff enthält, vorliegt, und diese Suspension in einem Schichtverdampfer aufgearbeitet wird.

Es sind bereits zahlreiche Verfahren zur Herstellung von Isocyanaten durch Umsetzung von primären Aminen mit Phosgen bekannt. Beispielsweise beschreibt US 3,140,305 ein Verfahren zur Herstellung von im wesentlichen reinem aromatischen Diisocyanat durch Umsetzung von Aminen mit Phosgen und anschließender Destillation der Umsetzungsprodukte.

US 3,892,634 beschreibt die Abtrennung von 2,4 Dimethylendiphenyldiisocyanat und 4,4'- Dimethylendiphenyldiisocyanat aus Polymethylenpolyphenylenpolyisocyanat mittels Schichtverdampfer. Auch FR 1 487 546, US 5,962,728 und US 3471543 beschreiben die Entfernung von monomerem Dimethylendiphenyldiisocyanat mittels Fallfilmverdampfer aus Polymethylenpolyphenylenpolyisocyanat.

RO 61 695 beschreibt die Reinigung von Dibenzyldiisocyanat, unter anderem mittels Fallfilmverdampfer.

US 4,118,286 beschreibt ein Verfahren zur Reduzierung der Säure aus Polymethylenpolyphenylenpolyisocyanat, wobei dieses unter anderem durch einen Fallfilmverdampfer geführt wird.

GB 1 083 910 beschreibt die Umsetzung von Polyisocyanaten mit Polyalkylen Polyethern zu Prepolymeren und anschließender Destillation in einem Fallfilmverdampfer.

Es ist weiterhin bekannt, dass die Verwendung eines hohen Phosgenüberschusses gegenüber den eingesetzten Aminogruppen zu hohen Selektivitäten bezüglich des hergestellten Isocyanats führt und somit einen entscheidenden Einfluss auf die Wirtschaftlichkeit des Verfahrens haben kann. Mit zunehmendem Verhältnis von Phosgen zu Aminogruppen steigt jedoch der Phosgen-Hold-Up der Anlage, wobei aufgrund der Giftigkeit von Phosgen ein möglichst geringer Phosgen-Hold-Up der Anlage angestrebt wird.

Aufgabe der Erfindung ist es daher, ein Verfahren zur Herstellung von Isocyanaten bereitzustellen, welches es gestattet, die Umsetzung mit möglichst geringen Ausbeuteverlusten bei gleichzeitig möglichst niedrigem Phosgen-Hold-Up durchzuführen.

Die Aufgabe konnte dadurch gelöst werden, dass der Reaktionsaustrag aus dem Phosgenierreaktor, welcher in Form einer Suspension vorliegt, die das herzustellende Isocyanat als Flüssigkeit und Carbamylchloride als Feststoff enthält, zur Aufarbeitung in einen Schichtverdampfer überführt wird. Die Lösung der Aufgabe war für den Fachmann überraschend, da bisher gegen den Einsatz von Feststoffen in Schichtverdampfer, insbesondere in Dünnschichtverdampfer, erhebliche Vorbehalte bestanden.

Gegenstand der Erfindung ist ein Verfahren zur Herstellung von Isocyanaten durch Umsetzung von primären Aminen mit Phosgen in einem Reaktor, **dadurch gekennzeichnet, dass** der Reaktionsaustrag in Form einer Suspension vorliegt, die das herzustellende Isocyanat als Flüssigkeit und Carbamylchloride als Feststoff enthält, und die Suspension in einem Schichtverdampfer aufgearbeitet wird.

Ferner ist Gegenstand der Erfindung die Verwendung von Schichtverdampfern zur Aufarbeitung von Reaktionsausträgen aus Phosgenierreaktoren, wobei die Reaktionsausträge in Form einer Suspension vorliegen, die das herzustellende Isocyanat als Flüssigkeit und Carbamylchloride als Feststoff enthält.

Für das erfindungsgemäße Verfahren kann ein beliebiges primäres Amin oder ein Gemisch aus zwei oder mehr dieser Amine eingesetzt werden. Bevorzugt werden aromatische Amine, insbesondere solche der Diaminodiphenylmethanreihe oder deren höheren Homologen eingesetzt. Beispiele sind Methylendiphenylamin (MDA; einzelne Isomere, Isomerengemisch und/oder Oligomere davon), Toluylendiamin (TDA), n-Pentylamin, 6-Methyl-2-heptanamin, Cyclopentylamin, R,S-1-Phenylethylamin, 1-Methyl-3-phenylpropylamin, 2,6-Xylidin, 2-(N,N-Dimethylamino)ethylamin, 2-(N,N-Diisopropylamino)ethylamin, C11-Neodiamin, 3,3'-Diaminodiphenylsulfon und 4-Aminomethyl-1,8-octandiamin. Bevorzugt verwendet werden MDA und TDA. Das Verfahren kann auch für aliphatische Amine verwendet werden. Hier ist die Verwendung für 1,6-Diaminohexan und für Isophorondiamin bevorzugt.

Das erfindungsgemäße Verfahren eignet sich entsprechend zur Herstellung von beliebigen Isocyanaten. Besonders vorteilhaft kann das Verfahren zur Herstellung von Methylen(diphenyldiisocyanat) (MDI) und Toluylendiisocyanat (TDI) angewandt werden.

Bei der Herstellung von Isocyanat durch Umsetzung von einem primären Amin mit Phosgen bildet sich gemäß nachstehendem Reaktionsschema in einem ersten schnellen Schritt intermediär Carbamylchloride, die sich im Geschwindigkeit bestimmenden, langsamen Schritt in einer Gleichgewichtsreaktion zu Isocyanaten und HCl zersetzten:

R-NH₂ + COCl₂ → R-NH-COCl + HCl ⇔ R -NCO + 2 HCl,

wobei R ein organischer Rest ist.
Ferner kann der entstehende Chlorwasserstoff mit Aminen zu Aminhydrochloriden (R-NH₃⁺Cl⁻) reagieren.

Die Umsetzung von Phosgen mit Diamin erfolgt im erfindungsgemäßen Verfahren in einem Reaktor. Hierbei kann es sich um alle üblichen, aus dem Stand der Technik bekannten Reaktoren handeln, die zur Phosgenierung, bevorzugt zur kontinuierlichen Phosgenierung, geeignet sind und den üblichen Drücken standhalten. Geeignete Materialien sind z.B. Metalle, wie Stahl, Silber oder Kupfer, Glas, Keramik oder homogenen oder heterogenen Gemischen daraus. Bevorzugt werden Stahlreaktoren verwendet.

Es können im allgemeinen die aus dem Stand der Technik bekannten Reaktorbautypen verwendet werden. Bevorzugt verwendet werden Rohrreaktoren, Kolonnen und Rührkessel.

Im erfindungsgemäßen Verfahren erfolgt die Vermischung der Reaktanten (Amin und Phosgen) in einer Mischeinrichtung, die sich durch eine hohe Scherung des durch die Mischeinrichtung geführten Reaktionsstromes auszeichnet. Bevorzugt werden als Mischeinrichtung eine Rotationsmischeinrichtung, eine Mischpumpe oder eine Mischdüse verwendet, die dem Reaktor vorangestellt ist. Besonders bevorzugt wird eine Mischdüse verwendet.

Das erfindungsgemäße Verfahren umfasst kontinuierliche, halbkontinuierliche und diskontinuierliche Verfahren. Bevorzugt sind kontinuierliche Verfahren.

Die Umsetzung von Amin mit Phosgen kann in der Flüssigphase oder in der Gasphase erfolgen. Bei den Umsetzungsbedingungen, wie beispielsweise Druck und Temperatur, handelt es sich im allgemeinen um die aus dem Stand der Technik bekannten Parameter.

Bevorzugt wird das erfindungsgemäße Verfahren in der Flüssigphase durchgeführt.

Bei einer Umsetzung von Amin mit Phosgen in der Flüssigphase kann dem erfindungsgemäßen Verfahren kann ein inertes Lösungsmittel beigesetzt werden. Dieses inerte Lösungsmittel ist üblicherweise ein organisches Lösungsmittel oder Gemische davon. Dabei sind Chlorbenzol, Dichlorbenzol, Trichlorbenzol, Toluol, Hexan, Diethylisophthalat (DEIP), Tetrahydrofuran (THF), Dimethylformamid (DMF), Benzol und deren Gemische bevorzugt. Besonders bevorzugt ist Chlorbenzol. Das inerte Lösungsmittel kann bevorzugt zu

Beginn der Umsetzung dem Amin zugesetzt werden. Das inerte Lösungsmittel wird üblicherweise in einer Menge von 5 bis 1000 Gew.-%, bevorzugt 50 bis 500 Gew.-%, bezogen auf die Menge an eingesetzten Amin, verwendet.

Das aus dem Reaktor austretende Umsetzungsgemisch (Reaktionsaustrag) liegt in Form einer Suspension vor. Diese Suspension enthält das herzustellende Isocyanat als Flüssigkeit und noch nicht zersetzte Carbamylchloride als Feststoffe. Gegebenenfalls enthält die aus dem Reaktor austretende Suspension weiterhin Aminhydrochloride und/oder Harnstoffe (R-NH-CO-NH-R) als Feststoffe.

Üblicherweise liegen in der Suspension (Reaktionsaustrag) Carbamylchloride in einer Menge von 0,01 bis 35 Gew.-%, bevorzugt 0,02 bis 30 Gew.-% bezogen auf die Menge der herzustellenden Isocyanats, vor. Dabei liegt im allgemeinen bei der Herstellung von MDI das Carbamylchlorid in einer Menge von 5 bis 35 Gew.-%, bevorzugt von 15 bis 30 Gew.-%, bezogen auf die Menge der herzustellenden Isocyanats, vor. Bei der Herstellung von TDI liegt im allgemeinen das Carbamylchlorid in einer Menge von 0,01 bis 30 Gew.-%, bevorzugt von 0,02 bis 20 Gew.-%, bezogen auf die Menge der herzustellenden Isocyanats, vor.

Weiterhin liegen im allgemeinen in der Suspension (Reaktionsaustrag) Aminhydrochloride in einer Menge von 0,01 bis 10 Gew.-%, bevorzugt 0,1 bis 5 Gew.-%, besonders bevorzugt 0,5 bis 3 Gew.-%, bezogen auf die Menge der herzustellenden Isocyanats, vor.

Schließlich liegen im allgemeinen in der Suspension (Reaktionsaustrag) Harnstoffe in einer Menge von 0,05 bis 15 Gew.-%, bevorzugt 0,5 bis 10 Gew.-%, besonders bevorzugt 1 bis 5 Gew.-%, bezogen auf die Menge der herzustellenden Isocyanats, vor.

Als Schichtverdampfer werden im Rahmen dieser Erfindung alle Vorrichtungen verstanden, bei denen die flüssige Phase des zu verdampfende Mediums als Schicht, bevorzugt als Film (dann entspricht der Schichtverdampfer einem Filmverdampfer) auf der Heizfläche aufgebracht wird, und an dieser rückvermischungsfrei zum Zwecke der Verdampfung entlang transportiert wird. Die Schicht wird auf der einen Seite durch die Heizfläche und auf der anderen Seite durch die gasförmige Phase begrenzt. Der Fall-filmverdampfer und der Dünnschichtverdampfer sind Beispiele für Schichtverdampfer.

Üblicherweise weist die im Schichtverdampfer zu verdampfende Flüssigkeit bzw. Suspension eine Schichtdicke von 0,01 bis 10 mm, bevorzugt von 0,1 bis 6 mm, besonders bevorzugt von 0,5 bis 3 mm auf.

In einer möglichen Ausführungsform können Dünnschichtverdampfer als Schichtverdampfer verwendet werden. Dünnschichtverdampfer sind im Stand der Technik bekannt und beispielsweise in Ullmanns Enzyklopädie beschrieben. Im allgemeinen dienen Dünnschichtverdampfer zur Verdampfung temperaturempfindlicher Substanzen von hochsiedenden Rückständen und für die Konzentration von temperaturlabilen Stoffen, wobei eine Flüssigkeit durch Abrieseln lassen, Einwirkung von Zentrifugalkraft, Wischern oder dergleichen zu dünnen Schichten auf beheizte Flächen aufgebracht wird.

Beispiele für geeignete Dünnschichtverdampfer sind beispielsweise Vorrichtungen, wobei der Flüssigkeitsfilm mechanisch erzeugt wird, beispielsweise sogenannte SAMBAY- und LUWA-Dünnschichtverdampfer sowie Sako-Dünnschichtverdampfer und ALFA-LAVAL-Centrithermverdampfer.

In einer bevorzugten Ausführungsform werden Schichtverdampfer verwendet, die keine bewegten Teile aufweisen. Beispiele hierfür sind Fallfilmverdampfer (auch als Fallstromverdampfer oder Fallschichtverdampfer bezeichnet) oder auch Wendelrohrverdampfer.

Bevorzugt werden im Rahmen dieser Erfindung Fallfilmverdampfer als Schichtverdampfer verwendet. Dabei kann die Wärmeübertragungsfläche in Form von Rohren oder Platten gestaltet sein. Bevorzugt werden zylindrische Rohre eingesetzt.

Üblicherweise wird der im erfindungsgemäßen Verfahren verwendete Schichtverdampfer, bevorzugt der verwendete Fallfilmverdampfer, bei Drücken von 0,5 mbar bis 25 bar, bevorzugt von 0,5 bar bis 20 bar, besonders bevorzugt von 1 bar bis 18 bar, betrieben.

Sofern es sich bei dem herzustellenden Isocyanat um TDI handelt, wird der im erfindungsgemäßen Verfahren verwendete Schichtverdampfer, bevorzugt der verwendete Fallfilmverdampfer, bei Drücken von 0,5 mbar bis 25 bar, bevorzugt von 0,5 bar bis 20 bar, besonders bevorzugt von 1 bar bis 18 bar, insbesondere von 2 bis 17 bar, betrieben.

Sofern es sich bei dem herzustellenden Isocyanat um MDI handelt, wird der im erfindungsgemäßen Verfahren verwendete Schichtverdampfer, bevorzugt der verwendete Fallfilmverdampfer, bei Drücken von 0,5 mbar bis 25 bar, bevorzugt von 0,5 bar bis 20 bar, besonders bevorzugt von 1 bar bis 18 bar, insbesondere von 1,5 bis 10 bar, betrieben.

Die Temperatur im Schichtverdampfer liegt üblicherweise zwischen 30°C und 300°C, bevorzugt zwischen 50°C und 200°C.

Die Verweilzeit der zu verdampfenden Flüssigkeit hängt von der eingestellten Temperatur ab. Üblicherweise beträgt die Verweilzeit im Schichtverdampfer von 5 Sekunden bis 20 Minuten, bevorzugt von 20 Sekunden bis 10 Minuten, besonders bevorzugt 40 Sekunden bis 400 Sekunden. Zur Erreichung der gewünschten Verweilzeit kann es sinnvoll sein, zwei oder mehr, bevorzugt zwei, Schichtverdampfer in Serie zu schalten. Die oben genannten Verweilzeiten beziehen sich dann auf die Summe der Verweilzeiten in den in Serie geschalteten Schichtverdampfern.

Die Strömungsführung für die Suspensionsphase und die Flüssigphase kann im Gleich- und im Gegenstrom erfolgen. Bevorzugt werden im verwendeten Schichtverdampfer flüssige und gasförmige Phase im Gegenstrom gefahren.

In einer möglichen Ausführungsform wird die Suspension (Reaktionsaustrag) in zwei, oder gegebenenfalls mehr als zwei, hintereinandergeschalteten Schichtverdampfern aufgearbeitet. In einer bevorzugten Ausführungsform arbeiten die zwei hintereinandergeschalteten Schichtverdampfer auf verschiedenen Druckstufen.

In einer bevorzugten Ausführungsform werden zwei Schichtverdampfer eingesetzt, wobei der erste Schichtverdampfer bei einem Druck von 0,5 bar bis 25 bar, bevorzugt von 0,5 bar bis 20 bar, besonders bevorzugt von 1 bar bis 18 bar, arbeitet und der zweite Schichtverdampfer einen Druck aufweist, der 0,01 bar bis 1 bar, bevorzugt 0,02 bar bis 0,5 bar, besonders bevorzugt 0,05 bis 0,2 bar geringer ist, als der Druck des ersten Schichtverdampfers.

Sofern mehr als zwei Schichtverdampfer in Serie geschaltet werden, so arbeitet der erste Schichtverdampfer bei einem Druck von 0,5 bar bis 25 bar, bevorzugt von 0,5 bar bis 20 bar, besonders bevorzugt von 1 bar bis 18 bar, arbeitet und jeder weitere Schichtverdampfer weist einen Druck auf, der um jeweils 0,01 bar bis 1 bar, bevorzugt 0,05 bar bis 0,2 bar geringer ist, als der Druck des vorhergehenden Schichtverdampfers.

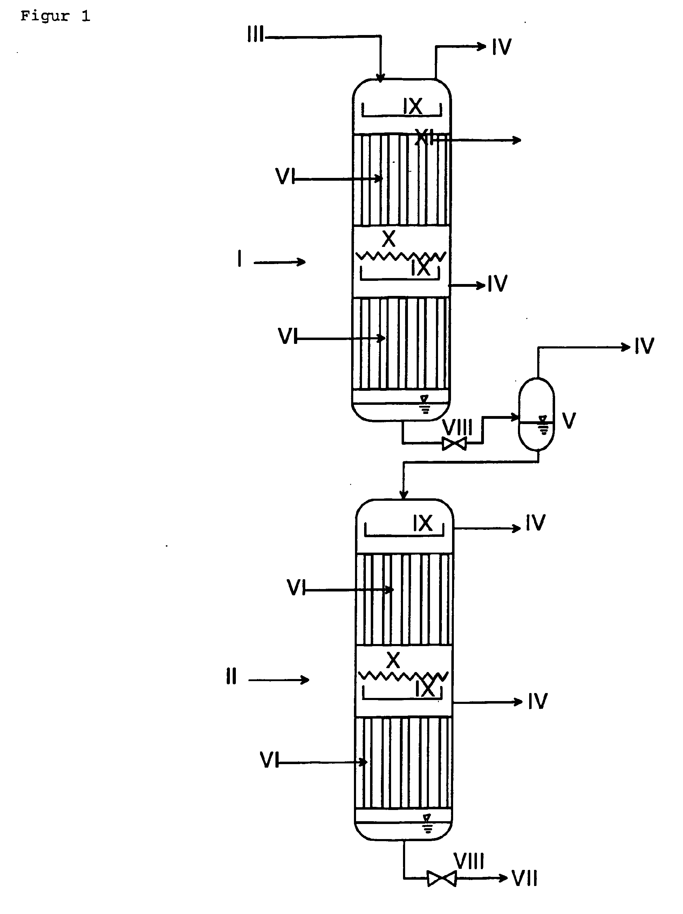

Eine bevorzugte Bauart für den Einsatz von Schichtverdampfern in der Aufarbeitung Reaktionsausträge ist in Figur 1 dargestellt:

Figur 1 veranschaulicht zwei hintereinandergeschalteten Fallfilmverdampfereinheiten I und II. In Figur 1 bedeutet:
- I: erster Fallfilmverdampfer
- II: zweiter Fallfilmverdampfer
- III: Zufuhr Reaktoraustrag
- IV: Abfuhr Gasphase
- V: Phasentrennbehälter
- VI: Rohrbündel mit mantelseitiger Beheizung
- VII: Entnahme Wertprodukt (flüssig)
- VIII: Druckreduziereinrichtung
- IX: Flüssigkeitsverteiler
- X: Flüssigkeitssammler
Fallfilmverdampfer I arbeitet bei einem Druck > 0,5 bar, bevorzugt 1 bis 5 bar absolut; II bei einem Druck, der unter dem von I liegt, jedoch bevorzugt > 3 mbar, ganz besonders bevorzugt > 50 mbar ist.

Auf den Kopf von I wird der suspensionsartige Reaktionsaustrag gegeben. Gegebenenfalls wird der Reaktionsaustrag vor der Aufgabe auf den Kopf von I noch einmal durch eine Druckreduziereinrichtung, bevorzugt ein Druckreduzierventil, geführt, und einem Phasentrennbehälter zugeführt, um den Druck auf den Betriebsdruck von I zu senken. Die aus dem Phasentrennbehälter entnommene Suspension gelangt dann nach Aufgabe auf den Kopf von I zunächst in einen Verteiler, wie z.B. einen Lochkastenverteiler und wird von dort auf Rohre eines Rohrbündels verteilt.

Die Rohre haben einen Außendurchmesser von 10 mm bis 200 mm, bevorzugt von 25 mm bis 80 mm. Die Länge der Rohre ist zwischen 0,5 m bis 15 m, bevorzugt 3 m bis 9 m. Die Anzahl der Rohre pro Fallfilmverdampfer beträgt üblicherweise 10 bis 10000, bevorzugt 100 bis 1000 Rohre.

Die Rohre haben beispielsweise einen Außendurchmesser von 38 mm, eine Wandstärke von 2,3 mm und eine Länge von 6000 mm. Es sind beispielsweise etwa 560 Rohre vorhanden. Am unteren Ausgang der Rohre wird die Suspension gesammelt und einem weiteren Verteiler zugeführt. Wieder folgt ein Rohrbündel wie oben angegeben.

Am unteren Teil dieses Rohrbündels folgt eine Standhaltung über die die Suspension über ein Ventil in ein Entspannungsgefäß zwecks Druckanpassung an II gegeben wird. II ist analog I konstruiert.

Die vorrangig flüssige Suspensionsphase hat in jeder Einheit Verteiler - Rohrbündel eine Verweilzeit von etwa 10 sec. Daraus folgt eine Gesamtverweilzeit von ca. 4 x10 sec = 40 sec. Durch Oberflächenstrukturierung kann die Verweilzeit von Feststoffen gegenüber der rein flüssigen Phase nochmals um etwa den Faktor drei erhöht werden. Damit ergibt sich eine maximale Verweilzeit in der Summe der Einheiten I und II von ca. 2 min.

Innerhalb von 40 sec Verweilzeit können Carbamylchloride bei einer Wandtemperatur von 150°C von anfänglich 0,1 bis 0,01 Gew.-% auf 1 ppm Gehalt, bezogen auf die Anfangsmenge an Carbamylchlorid abgebaut werden. Durch die Oberflächenstrukturierung ist sogar der Abbau von 10 bis 0,1 Gew.-% auf 1 ppm möglich.

Die flüssige Phase wird nun einer weiteren Aufarbeitung zugeführt, die je nach hergestelltem Isocyanat verschieden sein kann. I. a. wird nun im nächsten Schritt das inerte Lösungsmittel abgetrennt. Dies kann in einer Kolonne erfolgen oder wieder in einem Schichtverdampfer, bevorzugt einem Fallfilmverdampfer.

In einer bevorzugten Ausführungsform wird dem einen oder den mehreren verwendeten Schichtverdampfern eine Destillationskolonne nachgeschaltet.

In den Schichtverdampfern findet die Hauptspaltung des Isocyanates unter gleichzeitiger Phosgenabtrennung statt. Durch die Verwendung des Schichtverdampfers wird erfindungsgemäß eine Spaltung mit geringem Phosgen-Hold-Up erreicht. Der Endumsatz vom Carbamylchlorid, der eine relativ lange Zeit benötigt, wird dann durch Aufgabe der bereits phosgenarmen Lösung auf eine Destillationskolonne erreicht, in der genügend Hold-Up und damit Verweilzeit ohne größeren zusätzlichen Phosgen-Hold-Up erzeugt wird. Am Sumpf der Destillationskolonne wird die Isocyanatlösung abgezogen, am Kopf der Destillationskolonne die bei der restlichen Spaltung entstehende HCl und das restliche Phosgen.

Bei der Verwendung von mehreren Schichtverdampfern wird die Destillationskolonne üblicherweise dem im Verfahrensablauf zuletzt angeordneten Schichtverdampfer nachgeschaltet.

Die entnommenen Gasphasen, die hauptsächlich aus HCl und Phosgen, und zu einem geringeren Teil aus dem Lösungsmittel und in Spuren aus dem Isocyanat bestehen, werden einer weiteren Aufarbeitung zugeführt, wobei HCl und Phosgen getrennt werden. Evt. Spuren Isocyanat und/oder Lösungsmittel verbleiben im Phosgen. Das Phosgen wird in den Reaktionsteil zurückgeführt.

Das erfindungsgemäße Verfahren wird in einer Produktionsanlage durchgeführt, die einen Reaktor, in dem die Umsetzung von primären Aminen mit Phosgen stattfindet und mindestens einen Schichtverdampfer, dem der Reaktionsaustrag des Reaktors zugeführt wird, welcher in Form einer Suspension vorliegt, die das herzustellende Isocyanat als Flüssigkeit und Carbamylchloride als Feststoff enthält, umfasst.

Neben Reaktor und Schichtverdampfer umfasst die Produktionsanlage im allgemeinen weiterhin die aus dem Stand der Technik bekannten Reaktionsstufen, wie beispielsweise Vorlagebehälter, Mischvorrichtungen und Aufarbeitungsstufen.

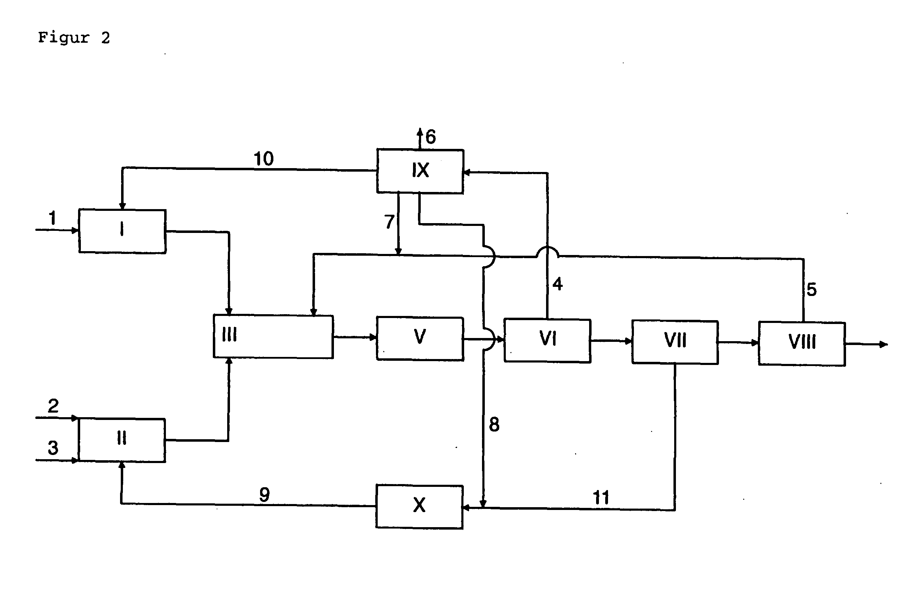

Eine bevorzugte Ausführungsform einer erfindungsgemäßen Produktionsanlage soll anhand eines allgemeinen Verfahrensschemas gemäß Figur 2 näher erläutert werden. In Figur 2 bedeutet:
- I: Phosgenvorlage
- II: Aminvorlage
- III: Mischvorrichtung
- V: Reaktor
- VI: Schichtverdampfer
- VII: Zweite Aufbereitungsvorrichtung
- VIII: Isocyanatvorlage
- IX: Phosgenaufarbeitung
- X: Lösungsmittelaufarbeitung
- 1: Zufuhr Phosgen
- 2: Zufuhr Amin
- 3: Zufuhr inertes Lösungsmittel
- 4: Abgetrennter Chlorwasserstoff und inertes Lösungsmittel
- 5: Rückgeführter Isocyanatstrom (optional)
- 6: Ausgetragener Chlorwasserstoff
- 7: Abgetrenntes Isocyanat
- 8, 11: Abgetrenntes inertes Lösungsmittel
- 9: Aufgearbeitetes inertes Lösungsmittel
- 10: Aufgearbeitetes Phosgen

Das Amin aus der Aminvorlage II und Phosgen, aus der Phosgenvorlage I werden in einer geeigneten Mischvorrichtung III vermischt. In einer optionalen Ausführungsform wird zusätzlich das Gemisch aus Amin und Phosgen mit rückgeführten Isocyanat als Lösungsmittel vermischt. Nach dem Vermischen wird das Gemisch in einen Reaktor V überführt. Ebenfalls sind Vorrichtungen verwendbar, die sowohl Misch- als auch Reaktionsvorrichtung darstellen, beispielsweise Rohrreaktoren mit angeflanschten Düsen.

Bei der Aufbereitungsvorrichtung VI handelt es sich um einen Schichtverdampfer in einer der vorstehend beschriebenen Ausführungsformen. Üblicherweise wird hier vom Isocyanatstrom Chlorwasserstoff und gegebenenfalls inertes Lösungsmittel und/oder geringe Anteile des Isocyanatstroms abgetrennt.

Bei der Aufbereitungsvorrichtung VII handelt es sich um eine übliche Destillationseinheit oder um einen weiteren Schichtverdampfer. In der zweiten Trennvorrichtung VII wird bevorzugt inertes Lösungsmittel abgetrennt und anschließend aufgearbeitet (X) und der Aminvorlage II wieder zugeführt.

Gegenstand der Erfindung ist neben dem erfindungsgemäßen Verfahren und der erfindungsgemäßen Produktionsanlage weiterhin die Verwendung von Schichtverdampfern zur Aufarbeitung von Reaktionsausträgen aus Phosgenierreaktoren, wobei die Reaktionsausträge in Form einer Suspension vorliegen, die das herzustellende Isocyanat als Flüssigkeit und Carbamylchloride als Feststoff enthält. Für die erfindungsgemäße Verwendung gelten entsprechend die vorstehend zum Verfahren und der Produktionsanlage dargelegten bevorzugten Ausführungsformen.

Die Verwendung von Schichtverdampfern zur Aufarbeitung von Reaktionsausträgen aus Phosgenierreaktoren führt zu folgenden Vorteilen:
- Der Phosgen-Hold-up der Gesamtanlage wird nachhaltig gesenkt, was ein wesentliches Sicherheitsmerkmal darstellt.
- Eine Produktionsanlage kann so konstruiert werden, dass die einzelnen schlanken Apparate individuell gekammert sind und eine Einhausung der Gesamtanlage entfallen kann.
- Die Investkosten werden gesenkt, d.h. die Wirtschaftlichkeit erhöht.

Der Schichtverdampfer hat dabei folgende Aufgaben:
- Schonendes Verdampfen von Phosgen und HCl.
- Bereitstellen einer weitgehend rückvermischungsfreien Strömung mit Überlagerung von chemischen Reaktionen,
- besonders der Zersetzung des Carbamylchlorids in Wertprodukt Isocyanat und HCl.
- Einbringen von Verdampfungs- und Reaktionswärme.
- Weitgehendes Abreichern von Phosgen, HCl, suspendierten Feststoffen und gelösten Reaktanten (etwa 0,5 % bis 0,01 ppm).

### Beispiel:

### Aufarbeitung von Reaktionsausträgen aus Phosgenierreaktoren

Ein Strom III aus der Vermischung von Phosgen mit einer Toluylendiamin-Lösung in Monochlorbenzol mit einem Massenstrom von 0,111 kg/s wurde nach Abtrennung der bei der Vermischung entstehenden Gasphase flüssig von oben in ein erstes Fallfilmverdampferrohr VI mit einem Innendurchmesser von 1,5 Zoll und einer Länge von 4 m geleitet. Der Strom hatte eine Temperatur von T = 70°C Er enthält 36 Massen-% Phosgen, 46 Massen-% MCB, 17 Massen-% Carbamylchlorid und 1 Massen-% HCl. Bei einem Druck von 4,5 bar absolut (abs) wurde über den Mantel an das Fallfilmverdampferrohr eine thermische Leistung von ca. 8,5 Kilowatt (KW) übertragen. Dampf- und Flüssigphase werden im Gegenstrom geführt. Die Flüssigphase wurde in einem Sammler X gesammelt und einem zweiten Fallfilmverdampferrohr VI mit gleichen Abmessungen wie dem ersten Fallfilmverdampferrohr von oben zugeführt. Auch hier wurde bei 4,5 bar (abs) eine thermische Leistung von ca. 8,5 KW übertragen. Dampf- und Flüssigphase werden im Gegenstrom geführt. Dabei wurde die Dampfphase des zweiten Fallfilmverdampferrohres nicht durch das erste Fallfilmverdampferrohr geleitet. Die flüssige Phase, die aus dem zweiten Fallfilmverdampferrohr abgezogen wird, enthielt bei einer Temperatur von T = 140°C 8 Massen-% Phosgen, 66 Massen-% Monochlorbenzol, 12 Massen-% TDI und 13-Massen-% Carbamylchlorid und wurde anschließend durch ein Ventil VIII in den Behälter V auf 2,5 bar entspannt. Der flüssige Abzug aus Behälter V wurde zur weiteren Aufarbeitung einem dritten Fallfilmverdampferrohr VI mit einer Länge von 6 m und einem Innendurchmesser von 1,5 Zoll zugeführt. Bei einem Druck von 2,5 bar (abs) wurde an das Fallfilmverdampferrohr eine thermische Leistung von ca. 3,9 KW übertragen. Dampf- und Flüssigphase wurden im Gegenstrom geführt. Die unten abgezogene Flüssigphase wurde im Sammler X gesammelt und von oben einem vierten Fallfilmverdampferrohr VI mit 6 m Länge und einem Durchmesser von 1,5 Zoll zugeführt. Bei einem Druck von 2,5 bar (abs) wurde an das Fallfilmverdampferrohr eine thermische Leistung von ca. 3,9 KW übertragen. Dampf- und Flüssigphase werden im Gegenstrom geführt. Dabei wird die Dampfphase des vierten Fallfilmverdampferrohres nicht durch das dritte Fallfilmverdampferrohr geleitet. Der flüssige Austrag aus dem vierten Fallfilmverdampferrohr enthielt bei einer Temperatur von T = 160°C noch 0,5 Massen-% Phosgen; 68 % Massen-MCB, 27 Massen-% TDI und 3 Massen-% Carbamylchlorid. Der flüssige Austrag wurde nach den üblichen Methoden zunächst der destillativen Phosgenentfernung in einer Destillationskolonne zugeführt und danach weiter destillativ bis zum reinen TDI aufbereitet. In der Destillationskolonne zur Phosgenentfernung zerfiel auch das restliche Carbamychlorid zu TDI und HCl.

## Patentansprüche

1. Verfahren zur Herstellung von Isocyanaten durch Umsetzung von primären Aminen mit Phosgen in einem Reaktor, **dadurch gekennzeichnet, dass** der Reaktionsaustrag in Form einer Suspension vorliegt, die das herzustellende Isocyanat als Flüssigkeit und Carbamylchloride als Feststoff enthält, und die Suspension in einem Schichtverdampfer aufgearbeitet wird.

2. Verfahren nach Anspruch 1, **dadurch gekennzeichnet, dass** es sich bei dem Schichtverdampfer um eine Vorrichtung handelt, die keine bewegten Teile aufweist.

3. Verfahren nach Anspruch 1 oder 2, **dadurch gekennzeichnet, dass** es sich bei dem Schichtverdampfer um einen Fallfilmverdampfer handelt.

4. Verfahren nach einem der Ansprüche 1 bis 3, **dadurch gekennzeichnet, dass** dem Schichtverdampfer eine Destillationskolonne nachgeschaltet ist.

5. Verfahren nach einem der Ansprüche 1 bis 4, **dadurch gekennzeichnet, dass** die Suspension in zwei oder mehreren hintereinandergeschalteten Schichtverdampfern, die auf verschiedenen Druckstufen arbeiten, aufgearbeitet wird.

6. Verfahren nach Anspruch 5, **dadurch gekennzeichnet, dass** der erste Schichtverdampfer bei einem Druck von 0,5 bar bis 25 bar arbeitet und der zweite Schichtverdampfer einen Druck aufweist, der 0,01 bar bis 1 bar geringer ist, als der Druck des ersten Schichtverdampfers.

7. Verfahren nach einem der Ansprüche 1 bis 6, **dadurch gekennzeichnet, dass** in der Suspension das Carbamylchlorid in einer Menge von 0,01 bis 35 Gew.-%, bezogen auf das Gewicht des herzustellenden Isocyanats, vorliegt.

8. Verfahren nach einem der Ansprüche 1 bis 7, **dadurch gekennzeichnet, dass** die Suspension als zusätzliche feste Bestandteile Aminhydrochloride und Harnstoffe enthält.

9. Verwendung von Schichtverdampfern zur Aufarbeitung von Reaktionsausträgen aus Phosgenierreaktoren, wobei die Reaktionsausträge in Form einer Suspension vorliegen, die das herzustellende Isocyanat als Flüssigkeit und Carbamylchloride als Feststoff enthält.

## Claims

1. A process for the preparation of isocyanates by reacting primary amines with phosgene in a reactor, wherein the reaction discharge is present in the form of a suspension which contains the isocyanate to be prepared, as a liquid, and carbamyl chlorides as a solid, and the suspension is worked up in a film evaporator.

2. A process as claimed in claim 1, wherein the film evaporator is an apparatus which has no moving parts.

3. A process as claimed in claim 1 or 2, wherein the film evaporator is a falling-film evaporator.

4. A process as claimed in any of claims 1 to 3, wherein a distillation column is connected downstream of the film evaporator.

5. A process as claimed in any of claims 1 to 4, wherein the suspension is worked up in two or more film evaporators which are arranged in series and operate at different pressure levels.

6. A process as claimed in claim 5, wherein the first film evaporator operates at from 0.5 to 25 bar and the second film evaporator has a pressure which is from 0.01 to 1 bar lower than the pressure of the first film evaporator.

7. A process as claimed in any of claims 1 to 6, wherein the carbamyl chloride is present in the suspension in an amount of from 0.01 to 35% by weight, based on the weight of the isocyanate to be prepared.

8. A process as claimed in any of claims 1 to 7, wherein the suspension contains amine hydrochlorides and ureas as additional solid components.

9. The use of film evaporators for working up reaction discharges from phosgenation reactors, the reaction discharges being present in the form of a suspension which contains the isocyanate to be prepared, as a liquid, and carbamyl chlorides as a solid.

## Revendications

1. Procédé de fabrication d'isocyanates par réaction d'amines primaires avec du phosgène dans un réacteur, **caractérisé en ce que** la sortie de la réaction se présente sous la forme d'une suspension, qui contient l'isocyanate à fabriquer sous forme liquide et des chlorures de carbamyle sous forme solide, et la suspension est traitée dans un évaporateur à couche.

2. Procédé selon la revendication 1, **caractérisé en ce que** l'évaporateur à couche est un dispositif qui ne comporte aucune partie agitée.

3. Procédé selon la revendication 1 ou 2, **caractérisé en ce que** l'évaporateur à couche est un évaporateur à film tombant.

4. Procédé selon l'une quelconque des revendications 1 à 3, **caractérisé en ce qu'**une colonne de distillation est connectée en aval de l'évaporateur à couche.

5. Procédé selon l'une quelconque des revendications 1 à 4, **caractérisé en ce que** la suspension est traitée dans deux évaporateurs à couche connectés en série ou plus, qui fonctionnent à des niveaux de pression différents.

6. Procédé selon la revendication 5, **caractérisé en ce que** le premier évaporateur à couche fonctionne à une pression de 0,5 bar à 25 bar et le second évaporateur à couche à une pression qui est inférieure de 0,01 bar à 1 bar à la pression du premier évaporateur à couche.

7. Procédé selon l'une quelconque des revendications 1 à 6, **caractérisé en ce que** le chlorure de carbamyle est présent dans la suspension en une quantité de 0,01 à 35 % en poids, par rapport au poids de l'isocyanate à fabriquer.

8. Procédé selon l'une quelconque des revendications 1 à 7, **caractérisé en ce que** la suspension contient des chlorhydrates d'amine et des urées en tant que constituants solides supplémentaires.

9. Utilisation d'évaporateurs à couche pour le traitement de sorties de réaction de réacteurs de phosgénation, les sorties de réaction se présentant sous la forme d'une suspension qui contient l'isocyanate à fabriquer sous forme liquide et des chlorures de carbamyle sous forme solide.
